Europäisches Patentamt

European Patent Office

Office européen des brevets

(19)

(11) EP 0 543 088 B1

(12) **EUROPÄISCHE PATENTSCHRIFT**

(45) Veröffentlichungstag und Bekanntmachung des Hinweises auf die Patenterteilung:
**10.07.1996 Patentblatt 1996/28**

(51) Int Cl.6: **C07D 339/04**, C07B 57/00, C07C 323/52

(21) Anmeldenummer: **92113729.5**

(22) Anmeldetag: **12.08.1992**

(54) **Herstellung und Verwendung von Salzen der reinen Enantiomere der alpha-Liponsäure**

Preparation and use of salts of the pure enantiomers of alpha-lipoic acid

Préparation et utilisation de sels des énantiomères purs de l'acide alpha-lipoique

(84) Benannte Vertragsstaaten:
**AT BE CH DE DK ES FR GB GR IE IT LI LU MC NL PT SE**

(30) Priorität: **16.11.1991 DE 4137773**

(43) Veröffentlichungstag der Anmeldung:
**26.05.1993 Patentblatt 1993/21**

(73) Patentinhaber: **ASTA Medica Aktiengesellschaft D-01277 Dresden (DE)**

(72) Erfinder:
- **Bethge, Horst W-6450 Hanau-Wolfgang (DE)**
- **Möller, Roland W-6451 Hammersbach (DE)**
- **Beisswenger, Thomas, Dr. W-6368 Bad Vilbel (DE)**
- **Huthmacher, Klaus, Dr. W-6460 Gelnhausen (DE)**
- **Blaschke, Gottfried, Prof. W-4400 Münster (DE)**
- **Scheidemantel, Ursula W-4400 Münster Hiltrup (DE)**

(56) Entgegenhaltungen:
- **JOURNAL OF THE AMERICAN CHEMICAL SOCIETY, Bd. 79, Nr. 24, 20. Dezember 1957, Washington, DC, US, Seiten 6483 - 6487**
- **CHEMICAL ABSTRACTS, vol. 94, no. 13, 30. M rz 1981, Columbus, Ohio, US; abstract no. 103722g, L.G. CHEBOTTAREVA, ET AL.: 'New method for separating racemic lipoic acid into antipodes' Seite 784 ;**
- **CHEMICAL ABSTRACTS, vol. 63, no. 13, 20. Dezember 1965, Columbus, Ohio, US; abstract no. 17809b, Spalte 17809 ;**

**Beschreibung**

Die Erfindung betrifft neue optisch aktive Salze aus α-Liponsäure und aus optisch aktivem α-Methylbenzylamin und ein Verfahren zur Herstellung der enantiomerenreinen α-Liponsäuren sowie der enantiomerenreinen Dihydroliponsäuren.

α-Liponsäure ist 1,2-Dithiolan-3-pentansäure (Thioctsäure).

α-Liponsäure besitzt als Coenzym der α-Ketosäuredehydrogenasen in Pflanzen und Tieren eine weite Verbreitung; die natürlich vorkommende Form besitzt die R-Konfiguration. Wenn im folgenden von "α-Liponsäure" die Rede ist, ist damit immer eine α-Liponsäure mit unbekannter stereochemischer Zusammensetzung gemeint.

α-Liponsäure ist pharmakologisch wirksam und weist antiphlogistische und antinociceptive (analgetische) sowie zytoprotektive Eigenschaften auf.

Von der α-Liponsäure sind eine Reihe von Salzen bekannt, so auch zum Beispiel Salze der α-Liponsäure mit optisch aktiven Basen wie zum Beispiel mit den basischen Aminosäuren Arginin und Lysin. (Spanisches Patent Nr. 313 056) Weder die Umsetzung von L-Arginin mit D,L-α-Liponsäure noch die Umsetzung von DL-Lysin mit D,L-α-Liponsäure ergeben trennbare diastereomere Salzpaare.

Bekannte Synthesen der enantiomerenreinen α-Liponsäure verlaufen immer über chirale Vorstufen, die im Laufe der Synthese gespalten werden. So wird in Walton, Wagner, Peterson, Holly und Folkers (J. Amer. chem. Soc. 76 (1954) Seite 4748ff) die Zwischenstufe 7-Carbethoxy-3-acetylthioheptansäure mit L-Ephedrin gespalten. Über die gleiche Zwischenstufe geht ein weiteres Verfahren, daß von den gleichen Autoren in J. Amer. Chem. Soc. 77 (1955) Seite 5144 publiziert wird. Das Japanische Patent 7970 beschreibt die Additionsverbindung von α-Liponsäure mit β-Cyclodextrin, ohne die Trennung in enantiomere Verbindungen zu erwähnen.

Acker und Wayne beschreiben in (J. Amer. Chem. Soc. 79 (24), 6483-6487 (1957)) die Darstellung von (+)-α-Liponsäure und (-)-α-Liponsäure durch Reduktion der enantiomerenreinen Vorstufe (+)-6,8-Dichloroctansäure bzw. (-)-6,8-Dichloroctansäure. Die enantiomerenreinen Dichloroctonsäuren werden durch Racematspaltung aus dem racemischen Gemisch (±) -6,8-Dichloroctansäure durch Umsetzung mit (-)-Ephedrin gewonnen.

Aufgabe der Erfindung ist die Herstellung von enantiomerenreinen Salzen der α-Liponsäure unter Verwendung einer optisch aktiven Hilfsbase und der anschließenden Freisetzung der reinen optischen Isomere der α-Liponsäure. Bei den reinen optischen Isomeren der α-Liponsäure (R- und S-Form, d.h. R-α-Liponsäure und S-α-Liponsäure) ist im Gegensatz zu dem Razemat das R-Enantiomere vorwiegend antiphlogistisch und das S-Enantiomere vorwiegend antinociceptiv wirksam (EP 0427247, 08.11.90).

Die Erfindung betrifft auch die Herstellung von Salzen der reinen optischen Isomere der α-Liponsäure mit den reinen optischen Isomeren des α-Methylbenzylamins. Hierbei wird so vorgegangen, daß in einem geeigneten Lösungsmittel die Isomeren bei erhöhter Temperatur, beispielsweise bei 30°C - 60°C, insbesondere bei 40°C aufgelöst werden und durch Kristallisation bei niedriger Temperatur, beispielsweise bei 10°C bis 30°C, insbesondere bei 25°C die reinen diastereomeren Salze isoliert werden. Als Lösungsmittel kommen neben Wasser in Frage: aliphatische und cycloaliphatische Kohlenwasserstoffe mit einer Kohlenstoffkettenlänge zwischen 3 und 10 Kohlenstoffatomen, aromatische Kohlenwasserstoffe, die flüssig sind, Ester aus aliphatischen oder cycloaliphatischen Carbonsäuren mit 2 bis 6 Kohlenstoffatomen und aliphatischen oder cycloaliphatischen Alkoholen mit 2 bis 6 Kohlenstoffatomen, aliphatische oder cycloaliphatische Alkohole mit 1 bis 6 Kohlenstoffatomen, Ether und Glycolether oder homogene Mischungen der genannten Lösungsmittel. Besonders bevorzugte Lösungsmittel sind Toluol, Essigsäureethylester und Cyclohexan. Vorzugsweise werden dabei zur Herstellung der diastereomeren Salze entweder das Gemisch aus der freien R-α-Liponsäure und S-α-Liponsäure direkt mit α-Methylbenzylamin umgesetzt oder auch das Gemisch aus R-α-Liponsäure und S-α-Liponsäure als Salz, z.B. Alkäli-oder Ammoniumsalz, mit einem Salz des α-Methylbenzylamins, z.B. Hydrochlorid oder Acetat, umgesetzt. Es ist auch möglich, ein Erdalkalisalz der α-Liponsäure mit einem Sulfat des α-Methylbenzylamins umzusetzen.

Überraschenderweise zeigen nun die diastereomeren Salzpaare Löslichkeitsunterschiede, so daß bei einer Umsetzung des Razemats der α-Liponsäure mit einem optisch reinen Isomer des α-Methylbenzylamins selektiv ein diastereomeres Salzpaar bevorzugt isoliert wird. Besonders vorteilhaft ist es, zur racemischen α-Liponsäure-Lösung nur 0,3 - 0.8. vorzugsweise jedoch 0,5 - 0.6 des molaren Äquivalents eines reinen Enantiomers des α-Methylbenzylamins zuzusetzen. Dabei kann selektiv ein diastereomeres Salzpaar bevorzugt isoliert werden. Das in der Mutterlauge jetzt stark angereicherte Enantiomer der α-Liponsäure kann durch Zusatz des anderen Enantiomers des α-Methylbenzylamins besonders angereichert erhalten werden. Diese Vorgehensweise eignet sich für ein kontinuierliches Herstellverfahren von sowohl R-α-Liponsäure als auch S-α-Liponsäure, wobei die beiden reinen Enantiomere weitgehend verlustfrei erhalten werden können. Durch Umkristallisation aus den reinen. bereits genannten Lösungsmitteln oder deren homogenen Mischungen können diese diastereomeren Salzpaare aufgereinigt werden, so daß schließlich reine Salzpaare vorliegen.

Die reinen Salzpaare aus R-α-Liponsäure und R-α-Methylbenzylamin beziehungsweise S-α-Liponsäure und S-α-Methylbenzylamin können durch Zusatz von Säuren, z.B. Mineralsäuren, gespalten werden und die reine R-α-Liponsäure oder die reine S-α-Liponsäure extraktiv isoliert werden.

Die Reinheit der optischen Isomere und der diastereomeren Salzpaare wurde mittels der spezifischen optischen Drehwerte bestimmt.

Weiterhin wurden durch Gaschromatographie an optisch aktiven Säulen relative Gehalte der optischen Isomere der α-Liponsäure mit einer Nachweisgrenze >0,5 % bestimmt. Die Werte werden als Enantiomerenüberschüsse (ee-Werte) angegeben.

Enantiomerenreine R-α-Liponsäure oder S-α-Liponsäure kann mit Diphenylmethylamin in ein stabiles, gut handhabbares Salz umgewandelt werden; die optische Reinheit der α-Liponsäure kann hier über den spezifischen optischen Drehwert ermittelt werden.

Die vorliegende Erfindung wird durch nachfolgende Beispiele näher erläutert.

Beispiel 1

20,6 g (100 mmol) R-α-Liponsäure wurden bei 40°C in 200 ml Toluol aufgelöst. Innerhalb von 5 min wurden 12,1 g (100 mmol) R-(+R-(+)-α-Methylbenzylamin zudosiert. Innerhalb von 2 h wurde auf 25°C abgekühlt. Der Niederschlag wurde abfiltriert und zweimal mit je 30 ml Toluol nachgewaschen. Das Salzpaar wurde im Vakuum bei 45°C getrocknet.

Man erhielt 32,4 g (99% d. Th.) R-α-Liponsäure-R-α-methylbenzylamin-Salz, $\alpha_D^{20}$ = + 74,0° (c=1; Ethanol), ee.: >99 % (GC) Löslichkeit in Toluol 0,09 % (25°C), in Wasser 1.16 % (25°C) Schmelzpunkt 109-115°C.

Beispiel 2

20,6 g (100 mmol) S-α-Liponsäure wurden bei 40°C in 200 ml Toluol aufgelöst. Innerhalb von 5 min wurden 12,1 g (100 mmol) R-α-Methylbenzylamin zudosiert. Innerhalb von 2 h wurde auf 25°C abgekühlt. Der Niederschlag wurde abfiltriert und zweimal mit je 30 ml Toluol nachgewaschen. Das Salzpaar wurde im Vakuum bei 45°C getrocknet.

Man erhielt 32,1 g (98 % d. Th.) S-α-Liponsäure-R-α-methylbenzylamin-Salz, $\alpha_D^{20}$ = - 59,2° (c = 1; Ethanol), ee.: >99 % GC. Löslichkeit in Toluol 0,12 % (25°C), in Wasser 1,41 % (25°C). Schmelzpunkt: 113-117°C.

Beispiel 3

20,6 g (100 mmol) S-α-Liponsäure wurden bei 40°C in 200 ml Toluol aufgelöst. Innerhalb von 5 min wurden 12,1 g (100 mmol) S-α-Methylbenzylamin zudosiert und, wie in Beispiel 1 beschrieben, aufgearbeitet.

Man erhielt 32,3 g (99 % d. Th.) S-α-Liponsäure-S-α-methylbenzylamin-Salz. $\alpha_D^{20}$ = -74,2° (c = 1; Ethanol), ee.: >99 % GC. Löslichkeit in Toluol 0,09 % (25°C) in Wasser 1,17 % (25°C). Schmelzpunkt: 109-115°C.

Beispiel 4

20,6 g (100 mmol) R-α-Liponsäure wurden bei 40°C in 200 ml Toluol aufgelöst. Innerhalb von 5 min wurden 12,1 g (100 mmol) S-α-Methylbenzylamin zudosiert und, wie in Beispiel 2 beschrieben, aufgearbeitet. Man erhielt 32,0 g (98 % d. Th.) R-α-Liponsäure-S-α-methylbenzylamin-Salz, $\alpha_D^{20}$ = +59,4°

(c = 1; Ethanol), ee.: >99 % GC. Löslichkeit in Toluol 0,12 (25°C) in Wasser 1,40 %. (25°C). Schmelzpunkt: 113-117°C.

Beispiel 5

Unter Lichtausschluß wurde die warme Lösung von 1,03 g (5 mmol) racemische α-Liponsäure in 75 ml mit $K_2CO_3$ getrocknetem Ethylacetat mit 0.303 g (2,5 mmol) R-alpha-Methylbenzylamin versetzt und auf Raumtemperatur, dann im Kühlschrank abgekühlt. Es kristallisierten 730 mg (89 %) aus, die hauptsächlich aus dem R-α-Liponsäure-R-α-methylbenzylamin-Salz bestanden. Dieses wurde zweimal aus jeweils 30 ml Ethylacetat umkristallisiert, wobei man 550 mg (67 %) aufgereinigtes Diastereomerensalz erhielt.

Zur Freisetzung der R-α-Liponsäure wurde das Salz in Wasser gelöst. die Lösung mit Ether überschichtet und unter Schütteln mit 0,1 N Salzsäure angesäuert. Man extrahierte die R-α-Liponsäure dreimal mit frischem Ether, wusch die vereinigten Etherphasen neutral und erhielt nach Trocknen und Abdampfen R-α-Liponsäure in praktisch quantitativer Ausbeute.

135 mg (0,654 mmol) der so erhaltenen R-α-Liponsäure wurden in 1 ml Ether gelöst und mit der Lösung von 152 mg (0,83 mmol) Diphenylmethylamin in 3,5 ml Ether versetzt. Der Niederschlag wurde aus Methanol (1 ml) Diisopropylether (5 ml) umkristallisiert. Man erhielt R-(α)-Liponsäure-diphenylmethylamin Salz mit einem Schmelzpunkt von 120°C, $\alpha_D^{20}$ = +51° (c=0,3; Pyridin).

Beispiel 6

20,6 g (100 mmol) racemische α-Liponsäure wurden bei 40°C in 200 ml Ethylacetat aufgelöst. Innerhalb von 5 min wurden 6,59 g (54 mmol) R-(+)-α-Methylbenzylamin zudosiert. Anschließend wurde innerhalb von 2 Stunden auf 25°C abgekühlt. Der Niederschlag wurde abfiltriert und zweimal mit je 35 ml Ethylacetat nachgewaschen. Das feuchte Salzpaar wurde viermal aus je 400 ml Ethylacetat umkristallisiert und anschließend im Vakuum bei 45°C getrocknet. Man erhielt 9,3 g Diastereomerensalz aus R-$\alpha$-Liponsäure und R-(+)-$\alpha$-Methylbenzylamin, $\alpha_D^{20} = +66,0°$ (c=1; Ethanol).

Das Salzpaar wurde in 300 ml Wasser bei 25°C suspendiert und mit 100 ml Cyclohexan versetzt. Unter Eiskühlung wurde mit 1 N Salzsäure langsam auf einen pH-Wert von 1 eingestellt. dann wurde auf 40°C erwärmt. Die Phasen wurden getrennt und die Wasserphase noch einmal mit 30 ml Cyclohexan nachextrahiert. Die vereinigten Cyclohexanextrakte wurden auf 5-10°C gekühlt und zur Kristallisation 5 Stunden bei dieser Temperatur nachgerührt. Der Niederschlag wurde abfiltriert. einmal mit 30 ml Cyclohexan nachgewaschen und bei 25°C im Vakuum getrocknet. Man erhielt 4,1 g (40 % d. Th.) R-($\alpha$)-Liponsäure, $\alpha_D^{28} = +104,1°$ (c=1; Benzol).

Beispiel 7

20,6 g (100 mmol) racemische $\alpha$-Liponsäure wurden bei 40°C in 200 ml Toluol aufgelöst. Innerhalb von 5 min wurden 6,59 g (54 mmol) R-(+)-$\alpha$-Methylbenzylamin zudosiert. Anschließend wurde innerhalb von 2 h auf 25°C abgekühlt, filtriert und der Niederschlag zweimal mit je 35 ml Toluol nachgewaschen. Das feuchte Salzpaar wurde viermal aus je 400 ml Toluol umkristallisiert und anschließend im Vakuum bei 45°C getrocknet. Man erhielt 10,6 g Diastereomerensalz, $\alpha_D^{20} = +72,5°$ (c=1; Ethanol).

Das Salzpaar wurde, wie in Beispiel 7 beschrieben, aufgespalten und die $\alpha$-Liponsäure aus Cyclohexan kristallin erhalten. Man erhielt 4,6 g (45 % d.Th.) R-$\alpha$-Liponsäure, $\alpha_D^{28} = +115,0°$ (c=1; Benzol) ee.: >99 % (GC).

Beispiel 8

A)
103 g (500 mmol) racemische $\alpha$-Liponsäure wurden bei 40°C in 1,0 l Toluol aufgelöst. Innerhalb von 5 min wurden 33,0 g (270 mmol) R-(+)-$\alpha$-Methylbenzylamin zugegeben. Anschließend wurde innerhalb von 2 h auf 25°C abgekühlt, filtriert und der Niederschlag zweimal mit je 150 ml Toluol nachgewaschen.

Das feuchte Salzpaar wurde, wie in Beispiel 8 angegeben, umkristallisiert. Nach Trocknen erhielt man 53,0 g (162 mmol) R-$\alpha$-Liponsäure-R-(+)-$\alpha$-Methylbenzylamin-Salz.

Die Mutterlaugen der Umkristallisation wurden weitgehend im Vakuum eingeengt und der Rückstand mit der Kristallisationsmutterlauge aufgenommen. Diese wurde mit 120 ml wäßriger Salzsäure extrahiert (Lösung R), wobei so viel Salzsäure zugegeben wurde, daß die wäßrige Phase einen pH-Wert von 1 aufwies. Die Toluolphase wurde anschließend mit zweimal 100 ml Wasser gewaschen.

B)
In der toluolischen $\alpha$-Liponsäurelösung aus A) wurden weitere 66,0 g (320 mmol) racemische $\alpha$-Liponsäure gelöst und bei 40°C mit 52,1 g (430 mmol) S-(-)-$\alpha$-Methylbenzylamin versetzt und anschließend innerhalb von 2 Stunden auf 25°C abgekühlt. Der Niederschlag wurde abfiltriert und zweimal mit je 150 ml Toluol nachgewaschen. Das feuchte Salzpaar wurde, wie in Beispiel 8 angegeben, umkristallisiert. Nach Trocknen erhielt man 91,7 g (280 mmol) S-$\alpha$-Liponsäure-S-(-)-$\alpha$-Methylbenzylamin-Salz. Die Mutterlaugen der Umkristallisation wurden weitgehend im Vakuum eingeengt und der Rückstand mit der Kristallisationsmutterlauge aufgenommen. Diese wurde mit 170 ml wäßriger Salzsäure extrahiert (Lösung S), wobei so viel Salzsäure zugegeben wurde, daß die wäßrige Phase einen pH-Wert von 1 aufwies. Die Toluolphase wurde anschließend mit zweimal 100 ml Wasser gewaschen.

C)
In der toluolischen $\alpha$-Liponsäurelösung aus B) wurden weitere 51,6 g (250 mmol) racemische $\alpha$-Liponsäure gelöst und bei 40°C mit 47.3 g (390 mol) R-(+)-$\alpha$-Methylbenzylamin versetzt und wie bei A) aufgearbeitet. Man erhielt 83,2 g (254 mmol) R-$\alpha$-Liponsäure-R-(+)-$\alpha$-Methylbenzylamin-Salz.

D)
In der toluolischen $\alpha$-Liponsäurelösung aus C) wurden weitere 51,6 g (250 mmol) racemische $\alpha$-Liponsäure gelöst und bei 40°C mit 47,3 g (390 mmol) S-(+S-(+)-$\alpha$-Methylbenzylamin versetzt und wie bei B) aufgearbeitet. Man erhielt 81,2 g (248 mmol) S-$\alpha$-Liponsäure-S-(-)-$\alpha$-Methylbenzylamin-Salz.

E)
136 g (416 mmol) R-$\alpha$-Liponsäure-R-(+)-$\alpha$-Methylbenzylamin-Salz wurden, wie in Beispiel 2 beschrieben, aufgespalten und dabei 60,9 g (295 mmol) R-$\alpha$-Liponsäure aus Cyclohexan kristallin erhalten. $\alpha_D^{20} = +119,0°$ (c = 1; Ethanol); $\alpha_D^{20} = +117,2°$ (c = 1,8; Benzol); ee.: >99 %; Fp. : 49-50°C. Die Mutterlauge kann für weitere Kristallisationsversuche verwendet werden. Die Hydrochlorid-Lösung des R-(+)-$\alpha$-Methylbenzylamins wurde mit den Lösungen R aus A) und C) vereinigt, mit Natronlauge auf einen pH-Wert von 13 eingestellt und mit Toluol extrahiert. Die Toluolphase wurde eingeengt und lieferte nahezu quantitativ das R-(+)-$\alpha$-Methylbenzylamin zurück.

F)
172 g (528 mmol) S-(-)-$\alpha$-Liponsäure-S-$\alpha$-Me-

thylbenzylamin Salz wurden, wie in Beispiel 2 beschrieben, aufgespalten und dabei 75,3 g (365 mmol) S-α-Liponsäure aus Cyclohexan kristallin erhalten. $\alpha_D^{20}$ = -119,4° (c = 1; Ethanol); Fp.: 49-50°C.

Die Hydrochlorid-Lösung des S-(-)-α-Methylbenzylamins wurde mit den Lösungen S aus B) und D) vereinigt, mit Natronlauge auf einen pH-Wert von 13 eingestellt und mit Toluol extrahiert. Die Toluolphase wurde eingeengt und lieferte nahezu quantitativ das S-(-)-α-tiethylbenzylamin zurück.

Beispiel 9 (nich erfindungsgemäß)

14,0 g Natriumhydroxid wurden in 130 ml Wasser vorgelegt. Bei Raumtemperatur wurden 20,6 g R-α-Liponsäure (100 mmol) eingetragen und bis zur Bildung einer klaren Lösung nachgerührt. Bei 20-25°C wurden dann 2,4 g (63 mmol) Natriumborhydrid in 35 ml Wasser gelöst innerhalb von 10 Minuten zugetropft. Dann wurde innerhalb von 1 Stunde auf 95°C aufgeheizt und 4 Stunden bei 95°C nachgerührt. Nach Abkühlen wurden 100 ml Toluol zugegeben und bei 10-15°C mit halbkonzentrierter Salzsäure auf einen pH-Wert von 1-1,5 eingestellt. Nach Phasentrennung wurde die Wasserphase noch einmal mit 50 ml Toluol extrahiert. Die vereinigten organischen Extrakte wurden im Vakuum eingeengt. Man erhielt 20,3 g (98 % der Theorie) an R-6,8-Dimercaptooctansäure. Die Destillation lieferte 18,5 g. (Sp 145-6°C, 0,3 mbar). $\alpha_D^{20}$: - 10,7° (c=1, Ethanol).

Beispiel 10 (nicht erfindungsgemäß)

21,0 g Natriumhydroxid wurden in 195 ml Wasser vorgelegt. Bei Raumtemperatur wurden 30,9 g S-α-Liponsäure eingetragen und bis zur Bildung einer klaren Lösung nachgerührt. Bei 20-25°C wurden dann 3,6 g (95 mmol) Natriumborhydrid in 50 ml Wasser gelöst innerhalb von 10 Minuten zugetropft. Dann wurde innerhalb von 1 Stunde auf 95°C aufgeheizt und 4 Stunden bei 95°C nachgerührt. Nach Abkühlen wurden 150 ml Toluol zugegeben und bei 10-15°C mit halbkonzentrierter Salzsäure auf einen pH-Wert von 1-1,5 eingestellt. Nach Phasentrennung wurde die Wasserphase noch einmal mit 75 ml Toluol extrahiert. Die vereinigten organische Extrakte wurden im Vakuum eingeengt. Man erhielt 30,3 g (97 % der Theorie) an S-6,8-Dimercaptooctansäure. Die Destillation lieferte 28,7 g (Sp. 145-6°C, 0,3 mbar). $\alpha_D^{20}$ : + 10,7° (c=1, Ethanol)

## Patentansprüche

1. Verfahren zur Trennung eines racemischen oder beliebig in Bezug auf die Enantiomeren zusammengesetzten Gemisches von R-α-Liponsäure und S-α-Liponsäure in seine optisch reinen Isomere, dadurch gekennzeichnet, daß man die Gemische mit einem optisch reinen Antipoden des α-Methylbenzylamins bei Temperaturen zwischen 20°C und 60°C in Toluol, Essigsäureethylester oder Cyclohexan umsetzt und die Mischung auf 10°C bis 30°C abkühlt und den Niederschlag isoliert und die Mutterlaugen weiter verarbeitet und die reinen diastereomeren Salzpaare durch Umsetzung mit anorganischen oder organischen Säuren spaltet.

2. Verfahren gemäß Anspruch 1,
dadurch gekennzeichnet, daß
das Verfahren kontinuierlich durchgeführt wird.

3. Salzpaar aus R-α-Liponsäure und R-(+)-α-Methylbenzylamin, Salzpaar aus R-α-Liponsäure und S-(-)-α-Methylbenzylamin, Salzpaar aus S-α-Liponsäure und R-α-Methylbenzylamin, und Salzpaar aus S-α-Liponsäure und S-(-)-α-Methylbenzylamin.

## Claims

1. Process for the separation of a mixture of R-α-lipoic acid and S-α-lipoic acid, which mixture is racemic or is of any composition with regard to the enantiomers, into the optically pure isomers thereof, characterised in that the mixtures are reacted with an optically pure antipode of α-methylbenzylamine at temperatures of between 20°C and 60°C in toluene, ethyl acetate or cyclohexane and the mixture is cooled to 10°C to 30°C and the precipitate is isolated and the mother liquors are further processed and the pure diastereomeric salt pairs are resolved by reaction with inorganic or organic acids.

2. Process according to claim 1, characterised in that the process is performed continuously.

3. Salt pair consisting of R-α-lipoic acid and R-(+)-α-methylbenzylamine, salt pair consisting of R-α-lipoic acid and S-(-)-α-methylbenzylamine, salt pair consisting of S-α-lipoic acid and R-α-methylbenzylamine and salt pair consisting of S-α-lipoic acid and S-(-)-α-methylbenzylamine.

## Revendications

1. Procédé de fractionnement d'un mélange racémique ou de composition quelconque concernant les énantiomères d'acide R-α-lipoïque et d'acide S-α-lipoïque en ses isomères optiquement purs, caractérisé en ce qu'on fait réagir les mélanges avec un antipode optiquement pur de l'α-méthylbenzylamine à des températures comprises entre 20°C et 60°C dans le toluène, l'acétate d'éthyle ou le cyclohexane et qu'on refroidit le mélange entre 10°C et 30°C et qu'on isole le précipité et qu'on traite à nouveau les lessives-mères et qu'on fractionne les paires de sels diastéréomères pures par réaction avec des acides minéraux ou organiques.

2. Procédé selon la revendication 1, caractérisé en ce qu'on réalise le procédé en continu.

3. Paire de sels d'acide R-α-lipoïque et de R-(+)-α-méthylbenzylamine, paire de sels d'acide R-α-lipoïque et de S-(-)-α-méthylbenzylamine, paire de sel d'acide S-α-lipoïque et de R-α-méthylbenzylamine, et paire de sels d'acide S-α-lipoïque et de S-(-)-α-méthylbenzylamine.